# EUROPEAN PATENT APPLICATION

(11) **EP 1 927 358 A1**
(43) Date of publication of application: **04.06.2008**
(21) Application number: 06782794.9
(22) Date of filing: 17.08.2006
(51) Int. Cl.: A61K 31/501, A61K 9/22, A61K 47/04, A61K 47/12, A61K 47/26, A61K 47/32, A61K 47/36, A61K 47/38, A61P 7/02

(54) **SUSTAINED-RELEASE PREPARATION**

(30) Priority: 23.08.2005 JP 2005241776
(71) Applicant: Nissan Chemical Industries, Ltd., Chiyoda-ku, Tokyo 101-0054 (JP)
(72) Inventor: SATO, Hirohiko c/o Nissan Chemical Industries, Ltd., Funabashi-shi Chiba 274-8507 (JP); YOKOYAMA, Tatsuro c/o Nissan Chemical Industries, Ltd., Funabashi-shi Chiba 274-8507 (JP); KANEZAKI, Shota c/o Nissan Chemical Industries, Ltd., Funabashi-shi Chiba 274-8507 (JP)
(74) Representative: Blodig, Wolfgang
(86) International application number: PCT/JP2006/316181
(87) International publication number: WO 2007/023729

(57) **Abstract**

It is intended to provide a sustained-release pharmaceutical preparation with absorbability not dependent on pH. The sustained-release preparation is **characterized by** containing 4-bromo-6-[3-(4-chlorophenyl)propoxy]-5-(3-pyridylmethylamino)-3(2H)-pyridazinone or a salt thereof, as a pharmaceutically active ingredient, and containing a hydrogel base and an organic acid.

## Description

### TECHNICAL FIELD

The present invention relates to a pH-independent sustained-release preparation which continuously releases a drug from a hydrogel layer.

### BACKGROUND ART

A sustained-release preparation is a highly useful preparation which is capable of reducing the number of doses and which controls a drug concentration in the blood to sustain the medicinal effect. For example, a drug required to be taken three times per day such as ibuprofen (half-life period: 2 hours) or phenylpropanolamine hydrochloride (half-life period: 4 hours), can be changed to dosing of twice per day. Further, the blood level of a drug having narrow ranges of the effective concentration and the side-effect development, such as theophyline, may be controlled to a prescribed concentration to make it possible to reduce the side effects and sustain the medicinal effect.

As one technique for preparation of such a sustained-release preparation, a hydrogel matrix preparation has been proposed which employs a water-soluble polymer as a sustained-release base. For example, Patent Document 1 discloses a hydrogel matrix preparation wherein nuclei made of a drug and a water-soluble polymer substance are coated with the same type of a water-soluble polymer substance. Further, Patent Document 2 discloses one obtained by compression molding of a drug, a hydrogel base and an enteric coating base. The hydrogel base is a water-soluble polymer capable of forming a hydrogel, and, for example, a carboxy vinyl polymer, hydroxyethyl cellulose, hydroxypropyl methylcellulose, hydroxyethyl methylcellulose, hydroxypropyl cellulose, methylcellulose, carboxymethyl cellulose, sodium carboxymethyl cellulose, polyethylene oxide, pullulan or sodium arginate is known.

On the other hand, the present inventors have heretofore conducted various studies on oral formulations of 4-bromo-6-[3-(4-chlorophenyl)propoxy]-5-(3-pyridylmethylamino)-3(2H)-pyridazinone (hereinafter referred to simply as Compound A) or its salt. As a result, it has been found that (1) the solubility in water decreases in the vicinity of pH4.0 as a boundary, and it becomes hardly soluble in water in the vicinity of the pH in the intestine, whereby the elution rate after oral administration is low, and (2) the absorbability in a dog is as low as a bioavailability of about 10% during fasting. Further, the present inventors have found that as a means to solve such problems relating to the elution rate and the absorbability, an organic acid such as citric acid, tartaric acid, malic acid, fumaric acid, malonic acid, succinic acid or maleic acid may be incorporated to the preparation, whereby it is possible to accomplish an improvement in the immediate elution and absorbability of Compound A or its salt (Patent Document 3).
Patent Document 1: JP-A-63-215620
Patent Document 2: JP-A-62-120315
Patent Document 3: JP-A-10-273440

### DISCLOSURE OF THE INVENTION

### OBJECT TO BE ACCOMPLISHED BY THE INVENTION

In view of the foregoing background art, it is an object of the present invention to provide a pH-independent sustained-release preparation of Compound A or its salt.

### MEANS TO ACCOMPLISH THE OBJECT

The present inventors have conducted an extensive study to accomplish the above object and as a result have found it possible to pH-independently control the elution of a pharmaceutically active substance containing Compound A or its salt from the preparation by adding a hydrogel base such as a carboxy vinyl polymer and an organic acid such as citric acid, tartaric acid, malic acid, fumaric acid, malonic acid, succinic acid or maleic acid as a solubility-assisting agent for the compound to the sustained-release matrix preparation containing Compound A or its salt.

The present invention has been made based on the above discovery and provides the following.
1. A sustained-release preparation comprising 4-bromo-6-[3-(4-chlorophenyl)propoxy]-5-(3-pyridylmethylamino)- 3(2H)-pyridazinone or a salt thereof, a hydrogel base and an organic acid.
2. The sustained-release preparation according to the above 1, wherein the salt of 4-bromo-6-[3-(4-chlorophenyl)propoxy]-5-(3-pyridylmethylamino)-3(2H)-pyridazinone is a hydrochloride.
3. The sustained-release preparation according to the above 1 or 2, wherein the hydrogel base is a carboxy vinyl polymer.
4. The sustained-release preparation according to the above 3, wherein the viscosity of a 0.2 mass% aqueous solution (20 rpm, 25°C) of the carboxy vinyl polymer is from 4,000 to 40,000 CPS.
5. The sustained-release preparation according to any one of the above 1 to 4, wherein the organic acid is at least one member selected from the group consisting of citric acid, tartaric acid, malic acid, fumaric acid, malonic acid, succinic acid and maleic acid.
6. The sustained-release preparation according to any one of the above 1 to 5, wherein one member or a mixture of at least two members selected from the group consisting of crystalline cellulose, lactose, sucrose, powder sugar, granular sugar, glucose, mannitol, sorbitol, starch, gum Arabic, dextrine, pullulan, light anhydrous silicic acid, low-substitution hydroxypropyl cellulose, sodium carboxymethyl cellulose, synthetic aluminum silicate and magnesium aluminometasilicate, is used as an excipient.
7. The sustained-release preparation according to any one of the above 1 to 6, wherein one member or a mixture of at least two members selected from the group consisting of magnesium stearate, calcium stearate, stearic acid, talc, light anhydrous silicic acid, colloidal silica, synthetic aluminum silicate, magnesium aluminometasilicate, calcium hydrogenphosphate and anhydrous calcium hydrogenphosphate, is used as a lubricant.
8. The sustained-release preparation according to any one of the above 1 to 7, wherein the content of 4-bromo-6-[3-(4-chlorophenyl)propoxy]-5-(3-pyridylmethylamino)-3(2H)-pyridazinone or a salt thereof, is from 1 to 10 mass%.
9. The sustained-release preparation according to any one of the above 1 to 8, wherein the amount of the hydrogel base is from 1 to 15 mass%.
10. The sustained-release preparation according to any one of the above 1 to 9, wherein the amount of the organic acid is from 5 to 20 mass%.

### EFFECTS OF THE INVENTION

The sustained-release preparation of the present invention is one which permits a drug having a pH-dependent solubility to elute without depending on the pH. When the preparation of the present invention is orally administered to a patient, the elution rate of the drug will not be changed by the pH of the digestive fluid in the digestive tract, whereby it is possible to minimize the variation of the blood drug concentration in an individual or among individuals. Further, it has an excellent effect such that by forming a hydrogel matrix layer, the time for maximum drug concentration in the blood can be prolonged without lowering the absorbability.

### BRIEF DESCRIPTION OF THE DRAWINGS

Fig. 1 shows the results of an elution test of a hydrochloride of Compound A in Comparative Example 1. ○: Japanese Pharmacopoeia Solution 1 (pH 1.2); ●: Japanese Pharmacopoeia Solution 2 (pH 6.8)+0.1% Tween 80
Fig. 2 shows the results of an elution test of a standard formulation in Comparative Example 2. ○: Japanese Pharmacopoeia Solution 1 (pH 1.2); ●: Japanese Pharmacopoeia Solution 2 (pH 6.8)+0.1% Tween 80
Fig. 3 shows the results of an elution test of a sustained-release preparation in Comparative Example 3 (a formulation having no citric acid incorporated in Example 1). O : Japanese Pharmacopoeia Solution 1 (pH 1.2); ●: Japanese Pharmacopoeia Solution 2 (pH 6.8)+0.1% Tween 80
Fig. 4 shows the results of an elution test of a sustained-release preparation of the present invention disclosed in Example 1. ○: Japanese Pharmacopoeia Solution 1 (pH 1.2); ●: Japanese Pharmacopoeia Solution 2 (pH 6.8) +0.1% Tween 80
Fig. 5 shows a plasma drug concentration when the preparation of the present invention in Example 1 or the standard formulation in Comparative Example 2 was orally administered. ○: Formulation of Comparative Example 2 (standard formulation); ●: Preparation of Example 1 (preparation of the present invention)

### BEST MODE FOR CARRYING OUT THE INVENTION

The hydrogel matrix sustained-release preparation of the present invention contains Compound A or its salt as an active ingredient. The salt of Compound A includes various salts so long as they maintain the medicinal effect of Compound A, but it is preferably a hydrochloride. Compound A or its salt may have an optional substituent so long as it maintains the medicinal effect. The sustained-release preparation of the present invention contains a hydrogel base and an organic acid in addition to Compound A or its salt. Further, it may suitably contain an excipient or a lubricant when it is made into an oral formulation such as tablets, capsules, granules, pills or powders.

The content of Compound A or its salt is not particularly limited so long as it is an amount within a range where the object of the present invention can be accomplished. However, it is preferably from 1 to 10 mass%, particularly preferably from 4 to 7 mass%, in the sustained-release preparation.

The hydrogel base to be used in the present invention is not particularly limited so long as it is pharmacologically acceptable and capable of accomplishing the object of the present invention. One member or a mixture of at least two members of such hydrogel bases may be employed. Specifically, one member or a mixture of at least two members selected from the group consisting of a carboxy vinyl polymer, hydroxyethyl cellulose, hydroxypropyl methylcellulose, hydroxyethyl methylcellulose, hydroxypropyl cellulose, methylcellulose, carboxymethyl cellulose, sodium carboxymethyl cellulose, polyethylene oxide, pullulan and sodium arginate may be used. Particularly preferred is a carboxy vinyl polymer. The viscosity of the hydrogel base to be used for the preparation of the present invention is not particularly limited. However, it is preferably from 3,000 to 80,000 CPS, particularly preferably from 4,000 to 40,000 CPS, as the viscosity of a 0.2 mass% aqueous solution (20 rpm, 25°C).

The amount of the hydrogel base is not particularly limited so long as it is an amount within which the object of the present invention can be accomplished. However, it is preferably from 1 to 15 mass%, particularly preferably from 1 to 10 mass%, in the sustained-release preparation.

The organic acid to be used in the present invention is not particularly limited so long as it is pharmacologically acceptable and capable of accomplishing the object of the present invention. One member or a mixture of at least two members of such organic acids may be used. Specifically, one member or a mixture of at least two members selected from the group consisting of citric acid, tartaric acid, adipic acid, ascorbic acid, malic acid, fumaric acid, malonic acid, succinic acid, maleic acid, aspartic acid, and glutamic acid may be used. As a preferred acid, citric acid, tartaric acid, malic acid, fumaric acid, malonic acid, succinic acid or maleic acid may, for example, be mentioned. As a particularly preferred acid, citric acid may be mentioned. The amount of the organic acid is not particularly limited so long as it is an amount within a range where the object of the present invention can be accomplished. However, it is preferably from 5 to 20 mass%, particularly preferably from 5 to 15 mass%, in the sustained-release preparation.

When the sustained-release preparation of the present invention is to be used for oral administration, it may be used in various formulations such as tablets, capsules, granules, pills or powders. Further, there is no particular limitation as to the administration form of the sustained-release preparation of the present invention, and other than the above-mentioned oral formulations, the administration form may be suitably selected as the case requires among parenteral formulations such as a suppository and an aerosol.

In a case where the sustained-release preparation of the present invention is made into an oral formulation such as tablets, capsules, granules, pills or powders, an excipient which may suitably be used for such a formulation, is not particularly limited so long as it is pharmacologically acceptable and capable of accomplishing the object of the present invention, and one member or a mixture of at least two members of such excipients may be used. Specifically, one member or a mixture of at least two members selected from the group consisting of crystalline cellulose, lactose, sucrose, powder sugar, granular sugar, glucose, mannitol, sorbitol, starch such as corn starch, wheat starch, rice starch, potato starch, hydroxypropyl starch, pregelatinized starch or partly pregelatinized starch, gum Arabic, dextrine, pullulan, light anhydrous silicic acid, low-substitution hydroxypropyl cellulose, sodium carboxymethyl cellulose, synthetic aluminum silicate and magnesium aluminometasilicate, may be used. The amount of the excipient is not particularly limited so long as it is an amount within a range where the object of the present invention can be accomplished. However, it is preferably from 53 to 93 mass%, particularly preferably from 70 to 85 mass%, in the sustained-release preparation.

Further, in a case where the sustained-release preparation of the present invention is made into an oral formulation such as tablets, capsules, granules or pills, a lubricant which may suitably be used for such a formulation is not particularly limited so long as it is pharmacologically acceptable and capable of accomplishing the object of the present invention. Specifically, one member or a mixture of at least two members selected from the group consisting of magnesium stearate, calcium stearate, stearic acid, talc, light anhydrous silicic acid, colloidal silica, synthetic aluminum silicate, magnesium aluminometasilicate, calcium hydrogenphosphate and anhydrous calcium hydrogenphosphate, may be used. The amount of the lubricant is not particularly limited so long as it is an amount within a range where the object of the present invention can be accomplished. However, it is preferably from 0.1 to 2 mass%, particularly preferably from 0.5 to 1 mass%, in the sustained-release preparation.

The sustained-release preparation of the present invention comprising the above-mentioned Compound A or its salt, the hydrogel base and the organic acid, may be prepared by various known methods. Oral formulations such as tablets, capsules, granules, pills and powders may be prepared by the respective production methods disclosed in Japanese Pharmacopoeia. For example, for the preparation of granules, a fluidized-bed granulation method, an agitation granulation method or an extrusion granulation method may, for example, be mentioned, and for the preparation of tablets, a granular compression method, a direct powder compression method or a casting method may, for example, be mentioned.

In a case where the sustained-release preparation of the present invention is made into an aerosol formulation, a dispersant and a propellant are not particularly limited so long as they are pharmacologically acceptable and capable of accomplishing the object of the present invention, and one member or a mixture of at least two members of such dispersants or propellants may be used. Specifically, as the dispersant, one member or a mixture of at least two members selected from the group consisting of soybean lecithins, egg-yolk lecithins, fatty acids such as oleic acid, linoleic acid and linolenic acid, sorbitans such as sorbitan trioleate and sorbitan monooleate, may be used. Further, as the propellant, a liquefied gas propellant or a compressed gas propellant may be used. As the liquefied gas propellant, specifically, a fluorinated hydrocarbon such as CFC-11, CFC-12, CFC-114, HCFC-123, HFC-134a or HFC-227, a liquefied petroleum or dimethyl ether, may, for example, be used. As the compressed gas, specifically, a soluble gas such as carbon dioxide gas or nitrous oxide gas, or an insoluble gas such as nitrogen gas may, for example, be used.

The aerosol formulation of the present invention may be prepared by a production method disclosed in Japanese Pharmacopoeia, and for example, a cooling filling method or a pressure filling method may, for example, be used.

In a case where the sustained-release preparation of the present invention is made into a suppository, the base is not particularly limited so long as it is pharmacologically acceptable and capable of accomplishing the object of the present invention, and one member or a mixture of at least two members of such bases may be used. Specifically, one member or a mixture of at least two members selected from the group consisting of bases which are solid at normal temperature, such as cacaobutter, serum laurinum, beef tallow or semi-synthetic hard fat, bee wax, myristic acid, stearic acid and palmitic acid, fats and oils which are liquid at normal temperature, such as coconuts oil, camellia oil, olive oil, palm kernel oil, soybean oil, sesame oil, corn oil, medium chain triglyceride, liquid paraffin, vaseline, lanoline, isopropyl myristate, and glycerol monostearate, surface active agents such as polyoxyethylene hardened castor oil, sorbitan fatty acid ester, polyethylene glycol fatty acid ester, polyoxyethylene sorbitan fatty acid ester, dextrin fatty acid ester, and sucrose fatty acid ester, and higher alcohols such as stearyl alcohol and cetyl alcohol, may be used.

The suppository of the present invention may be prepared by a production method disclosed in Japanese Pharmacopoeia, and for example, a fusing method or a cold press method may be employed. The shape of the suppository is not particularly limited so long as it can be administered as a suppository, but it may, for example, be conical, spindle-shaped, spherical or oval.

### EXAMPLES

Now, the present invention will be described in detail with reference to Examples and Comparative Examples, but it should be understood that the present invention is by no means thereby restricted.

### COMPARATIVE EXAMPLE 1

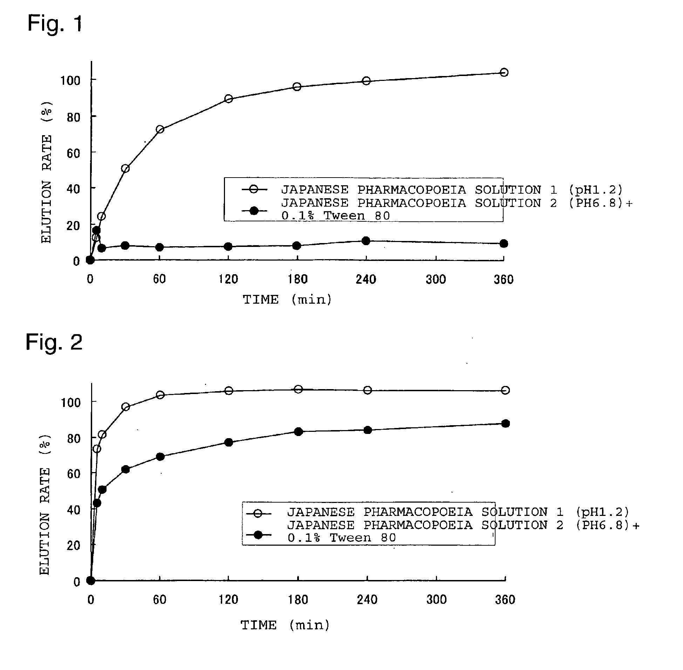

The results of an elution test of a hydrochloride of Compound A, as a simple substance, are shown in Fig. 1. The hydrochloride of Compound A showed a substantial difference in the elution rate as between Japanese Pharmacopoeia Disintegration Test Solution 1 and Solution 2 containing 0.1% Tween 80. Further, in Japanese Pharmacopoeia Disintegration Test Solution 2 +0.1% Tween 80, the elution rate did not reach even 20% even after 6 hours, thus indicating that it is hardly soluble in the vicinity of the pH in the intestine. Thus, the results in Fig. 1 show that the elution of the hydrochloride of Compound A is influenced substantially by the liquid composition and the pH in the elution environment.

### COMPARATIVE EXAMPLE 2

Based on the prescription in Table 1, a standard formulation (tablet) of the hydrochloride of Compound A was prepared. The mass of a tablet was adjusted to be 150 mg (corresponding to 6 mg of the hydrochloride of Compound A).

**TABLE 1 PRESCRIPTION**

| | |
|---|---|
| Hydrochloride of Compound A | 6.0 g |
| Crystalline cellulose | 108.9 g |
| Low substitution hydroxypropyl cellulose | 15.0 g |
| Hydroxypropyl methylcellulose | 4.5 g |
| Citric acid | 15.0 g |
| Magnesium stearate | 0.6 g |
| Total | 150.0 g |

### The test conditions are as follows.

Test solutions: Japanese Pharmacopoeia Disintegration Test Solution 1 (pH 1.2) 900 mL, Japanese Pharmacopoeia Disintegration Test Solution 2 900 mL (pH 6.8)+0.1% Tween 80
Paddle rotational speed: 50 rpm
Liquid temperature: 37°C

The results of an elution test of the standard formulation are shown in Fig. 2. The standard formulation showed a substantial difference in the elution rate as between Japanese Pharmacopoeia Disintegration Test Solution 1 and Solution 2 containing 0.1% Tween 80. Further, in each test solution, the initial elution was observed to be remarkably fast. Thus, the results in Fig. 2 shows that the elution of the standard formulation is influenced substantially by the liquid composition and the pH in the elution environment, and is of an immediate elution type.

### COMPARATIVE EXAMPLE 3

For the purpose of making the role of citric acid incorporated in the present invention clear, the following formulation was prepared. This formulation corresponds to the formulation having no citric acid incorporated in Example 1.

**TABLE 2 PRESCRIPTION**

| | |
|---|---|
| Hydrochloride of Compound A | 6.0 g |
| Carboxy vinyl polymer | 15.0 g |
| Crystalline cellulose | 128.0 g |
| Magnesium stearate | 1.0 g |
| Total | 150.0 g |

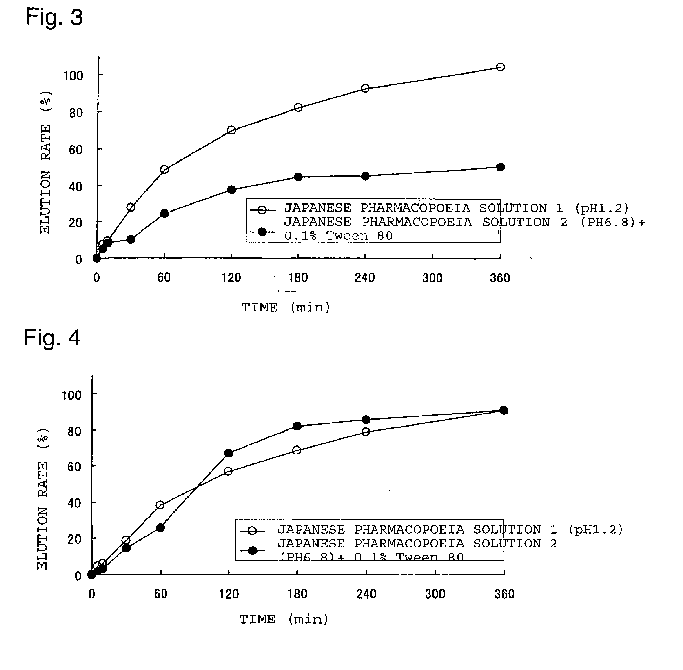

Based on the above prescription, the hydrochloride of Compound A and crystalline cellulose were mixed and granulated by high-performance agitation granulation. Air-circulating constant temperature drying was carried out at 60°C for from 16 to 18 hours, followed by sieving with 30 mesh. To the sieved product, the carboxy vinyl polymer and magnesium stearate were further added and mixed, followed by tableting. The mass of a tablet was adjusted to be 150 mg (corresponding to 6 mg of the hydrochloride of Compound A). With respect to this tablet, an elution test of the hydrochloride of Compound A was carried out in the same manner as described above to obtain the results in Fig. 3.

In this test, although the immediate elution was suppressed, a substantial difference in the elution rate was observed as between Japanese Pharmacopoeia Disintegration Test Solution 1 and Solution 2 containing 0.1% Tween 80. Thus, the results in Fig. 3 show that although the sustained-release effect by the carboxy vinyl polymer is accomplished, the elution is influenced by the liquid composition and the pH in the elution environment due to the pH-dependent solubility of Compound A.

### EXAMPLE 1

A sustained-release preparation (tablet) of the present invention was prepared as follows.

**TABLE 3 PRESCRIPTION**

| | |
|---|---|
| Hydrochloride of Compound A | 6.0 g |
| Carboxy vinyl polymer | 15.0 g |
| Crystalline cellulose | 113.0 g |
| Citric acid | 15.0 g |
| Magnesium stearate | 1.0 g |
| Total | 150.0 g |

Based on the above prescription, the hydrochloride of Compound A, crystalline cellulose and citric acid were mixed and granulated by high-performance agitation granulation. Air-circulating constant drying was carried out at 60°C for from 16 to 18 hours, followed by sieving with 30 mesh. To the sieved product, the carboxy vinyl polymer and magnesium stearate were further added and mixed, followed by tableting. The mass of a tablet was adjusted to be 150 mg (corresponding to 6 mg of the hydrochloride of Compound A). With respect to this tablet, an elution test of the hydrochloride of Compound A was carried out in the same manner as described above to obtain the results in Fig. 4.

In this Example, no difference in the elution rate of the hydrochloride of Compound A was observed between Japanese Pharmacopoeia Solution 1 and Solution 2, thus indicating that the elution is not influenced by the liquid composition or the pH in the elution test environment and that immediate elution is suppressed, and constant 0-order release is accomplished up to 6 hours.

### EXAMPLE 2

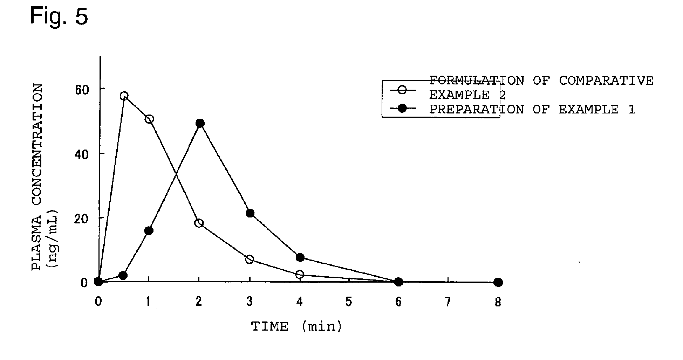

In order to confirm the sustained-release effect of the present invention, to three beagles fasted overnight, the standard formulation of Comparative Example and the sustained-release preparation of Example 1 were respective orally administered so that the hydrochloride of Compound A would be 30 mg. After the administration, from the forelimb vein, the blood was sampled with time, and the sampled blood was centrifugally separated, whereupon Compound A in plasma was measured by a high performance liquid chromatography. The results are shown in Fig. 5. This Example shows that as compared with the standard formulation, the sustained-release preparation of the hydrochloride of Compound A remarkably prolongs the time for maximum drug concentration in the blood without lowering the absorbability, thus indicating a sustained-release effect.

### INDUSTRIAL APPLICABILITY

The sustained-release preparation of the present invention is one which permits a drug having a pH dependent solubility to elute without depending on the pH. Elution of the drug will thereby be less influenced by the pH or composition of the eluent, and when it is orally administered to a patient, the elution rate of the drug will not be changed by the pH of the digestive fluid in the digestive tract, whereby it is possible to minimize the variation of the blood drug concentration in an individual or among individuals. Further, by forming a hydrogel matrix layer, it has been made possible to prolong the time for maximum drug concentration in the blood without lowering the absorbability.

The entire disclosure of Japanese Patent Application No. 2005-241776 filed on August 23, 2005 including specification, claims, drawings and summary is incorporated herein by reference in its entirety.

## Claims

1. A sustained-release preparation comprising 4-bromo-6-[3-(4-chlorophenyl)propoxy]-5-(3-pyridylmethylamino)-3(2H)-pyridazinone or a salt thereof, a hydrogel base and an organic acid.

2. The sustained-release preparation according to Claim 1, wherein the salt of 4-bromo-6-[3-(4-chlorophenyl)propoxy]-5-(3-pyridylmethylamino)-3(2H)-pyridazinone is a hydrochloride.

3. The sustained-release preparation according to Claim 1 or 2, wherein the hydrogel base is a carboxy vinyl polymer.

4. The sustained-release preparation according to Claim 3, wherein the viscosity of a 0.2 mass% aqueous solution (20 rpm, 25°C) of the carboxy vinyl polymer is from 4,000 to 40,000 CPS.

5. The sustained-release preparation according to any one of Claims 1 to 4, wherein the organic acid is at least one member selected from the group consisting of citric acid, tartaric acid, malic acid, fumaric acid, malonic acid, succinic acid and maleic acid.

6. The sustained-release preparation according to any one of Claims 1 to 5, wherein one member or a mixture of at least two members selected from the group consisting of crystalline cellulose, lactose, sucrose, powder sugar, granular sugar, glucose, mannitol, sorbitol, starch, gum Arabic, dextrine, pullulan, light anhydrous silicic acid, low-substitution hydroxypropyl cellulose, sodium carboxymethyl cellulose, synthetic aluminum silicate and magnesium aluminometasilicate, is used as an excipient.

7. The sustained-release preparation according to any one of Claims 1 to 6, wherein one member or a mixture of at least two members selected from the group consisting of magnesium stearate, calcium stearate, stearic acid, talc, light anhydrous silicic acid, colloidal silica, synthetic aluminum silicate, magnesium aluminometasilicate, calcium hydrogenphosphate and anhydrous calcium hydrogenphosphate, is used as a lubricant.

8. The sustained-release preparation according to any one of Claims 1 to 7, wherein the content of 4-bromo-6-[3-(4-chlorophenyl)propoxy]-5-(3-pyridylmethylamino)-3(2H)-pyridazinone or a salt thereof, is from 1 to 10 mass%.

9. The sustained-release preparation according to any one of Claims 1 to 8, wherein the amount of the hydrogel base is from 1 to 15 mass%.

10. The sustained-release preparation according to any one of Claims 1 to 9, wherein the amount of the organic acid is from 5 to 20 mass%.
